# EUROPEAN PATENT APPLICATION

(11) **EP 4 424 700 A1**
(43) Date of publication of application: **04.09.2024**
(21) Application number: 22887617.3
(22) Date of filing: 26.10.2022
(51) Int. Cl.: C07K 7/08, A61K 38/00, A61P 35/00

(54) **NOVEL PEPTIDE TARGETING DENDRITIC CELLS, AND COMPOSITION FOR TREATING CANCER COMPRISING SAME**

(30) Priority: 28.10.2021 KR 20210146081
(71) Applicant: Industry Foundation of Chonnam National University, Gwangju 61186 (KR)
(72) Inventor: RHEE, Joon Haeng, Gwangju 61903 (KR); LEE, Shee Eun, Gwangju 61198 (KR); VIVEK, Verma, Hwasun-gun Jeollanam-do 58114 (KR); PUTH, Sao, Hwasun-gun Jeollanam-do 58114 (KR); HONG, Seol Hee, Gwangju 61508 (KR)
(74) Representative: Plougmann Vingtoft a/s
(86) International application number: PCT/KR2022/016492
(87) International publication number: WO 2023/075421

(57) **Abstract**

The present invention relates to a novel peptide targeting dendritic cells in the body, and a composition which is for treating cancer, and comprises the peptide. Specifically, the present invention relates to a composition for treating or preventing cancer, the composition comprising: a novel peptide targeting dendritic cells in the body; a tumor antigen; and flagellin. In addition, the novel peptide targeting dendritic cells in the body, the tumor antigen, and the flagellin according to the present invention may be linked by a single peptide or included in respective peptides.

## Description

### Technical Field

The present disclosure relates to a novel peptide targeting dendritic cells in vivo, and a composition including same for the treatment of cancer. Specifically, the present disclosure is concerned with a novel peptide that targets dendritic cells in vivo, and a composition including the novel peptide, a tumor antigen, and flagellin for prevention or treatment of cancer. Furthermore, the novel peptide of the present disclosure that targets dendritic cells in vivo, the tumor antigen, and the flagellin may be linked to form a single peptide or may be included as individual peptides.

### Background Art

Conventional cancer treatment methods include surgery, radiation therapy, and chemotherapy using anti-cancer drugs to remove cancer cells as much as possible. These methods, although used relatively widely in cancer treatment, have not established themselves as fundamental treatments for cancer because they have side effects and difficulty in leading to a complete cure. Especially for metastatic or recurrent cancer, surgery is mostly not feasible, and many instances show resistance to chemical treatments. Therefore, there is an urgent need to develop new treatments for such cancer patients.

To overcome the limitations of conventional cancer treatments, recent research has been actively pursued into anti-cancer immunotherapy, and its clinical use has been approved both domestically and abroad. Anti-cancer immunotherapy utilizes the properties of the body's immune cells to increase immune activity against cancer cells or suppress the methods by which cancer cells evade attacks from immune cells. This immunotherapy encompasses immune cell therapy, immune checkpoint inhibitors, therapeutic cancer vaccines, and therapeutic antibodies.

Therapeutic cancer vaccines include: cancer vaccines using native cancer tissues (intact tumor cells), cancer cell lysates derived from cancer cell lines, or tumor antigens; and dendritic cell (DC)-based cancer vaccines that utilize dendritic cells (DC) generated by exposing cancer cell-derived antigens or cancer cell lysates.

The cancer treatments using dendritic cells under recent research and development are more patient-oriented than any other existing treatments and can offer long-term efficacy through memory immunity, making them not only very effective in preventing metastasis or recurrence of the same cancer, but also safe. Therefore, they are anticipated as a new anti-cancer immunotherapy and are being developed as therapeutic vaccines for various types of cancer. In particular, dendritic cell cancer vaccines have the potential to be used as a treatment to prevent metastasis and recurrence after surgical removal of the primary cancer, so they will have significant competitiveness in the cancer treatment market.

Dendritic cells (DC) are antigen-presenting cells of the mammalian immune system and have branch-like projections that resemble membranes or spines. The primary function of dendritic cells is to process antigen materials and present them to T cells of the immune system. Thus, dendritic cells are essential antigen-presenting cells that activate naive T lymphocytes, acting as messengers between the innate and adaptive immune systems. The efficacy of cancer treatments utilizing the immune system has been demonstrated in many animal experiments so far, and the identification of tumor-specific antigens recognized by human T lymphocytes is promoting the development of immunotherapy.

Research has been made into various anti-cancer vaccine strategies targeting dendritic cells. Some are designed to manipulate dendritic cells in vitro, while others are based on stimulating dendritic cells in vivo. In the former approach, dendritic cells are differentiated from blood cells collected from the patient. That is, they are cultured and matured ex vivo with tumor antigen peptides, tumor lysates, apoptotic tumor cells, or heat shock proteins extracted from autologous tumors, and are ultimately re-infused into the patient. However, recent research suggests that using immature dendritic cells as a cellular therapy could potentially activate cancer further, indicating the importance of using sufficiently mature dendritic cells. The cost of creating this personalized treatment is also a drawback. In the latter approach, stimulation of the dendritic cells occurs after introducing peptides, proteins, irradiated tumor cells, or other viruses containing antigenic peptides targeting dendritic cells into the patient. However, the degree of dendritic cell activation appears to be a delicate factor because it influences the ability to effectively activate cytotoxic T lymphocytes.

Flagellin is a constituent unit protein that makes up the filament of flagella. Flagellin is systematically combined to form the filament that allows bacteria to be motile. Flagellin is a substance that stimulates pattern recognition receptors and has been studied as a target for the development of vaccine carrier proteins or vaccine adjuvants. Fusion proteins of flagellin with an antigen have been proven effective as experimental vaccines against diverse infectious diseases, including pneumonia, West Nile fever, malaria, tuberculosis, and bacterial periodontal diseases. Flagellin-activated TLR5 has been reported to protect hematopoietic cells and gastrointestinal tissues from radiation and to affect cancer cell survival and growth. Moreover, it is disclosed in Korean Patent No. 10-0795839 issued to the present inventors that FlaB, a component of Vibrio bacteria causing septicemia, acts on the host cell's Toll-like receptor 5 to induce a strong immunomodulatory effect, thus demonstrating its excellent efficacy as a mucosal vaccine adjuvant.

In the present disclosure, a discovery was made of a novel peptide targeting dendritic cells in vivo through biopanning, and a recombinant integrated polypeptide vaccine containing the novel peptide targeting dendritic cells, a tumor antigen, and flagellin was developed and led to a composition with better effects in cancer treatment.

### [Related Art Documents]

### [Patent Literature]

(Patent Literature 1) Korean Patent No. 10-0795839

### [Non-Patent Literature]

(Non-Patent Literature 1) Lee SE, Kim SY, Jeong BC, Kim YR, Bae SJ, Ahn OS, et al. A bacterial flagellin, Vibrio vulnificus FlaB, has a strong mucosal adjuvant activity to induce protective immunity. Infect Immun. 2006;74(1) :694-702.

### Detailed Description of the Invention

### Technical Problem

An aspect of the present disclosure is to provide a novel peptide targeting dendritic cells in vivo.

Another aspect of the present disclosure is to provide a peptide including a novel peptide targeting dendritic cell, a tumor antigen, and flagellin for treating or preventing cancer.

A further aspect of the present disclosure is to provide a pharmaceutical composition including a novel peptide targeting dendritic cells in vivo, a tumor antigen, and flagellin for treating or preventing cancer.

A still further aspect of the present disclosure is to provide an immune composition including a novel peptide targeting dendritic cells in vivo, a tumor antigen, and flagellin for treating or preventing cancer.

### Technical Solution

The present disclosure provides a dendritic cell-targeting peptide for treating or preventing cancer.

Also, the present disclosure provides a polynucleotide including a nucleic acid sequence coding for the peptide.

The dendritic cell-targeting peptide for treating or preventing cancer may include an amino acid sequence selected from the group consisting of SEQ ID NOS: 1 to 11, any one amino acid sequence preferably selected from the group consisting of SEQ IS NOS: 3, 4, 6, 9, and 10, more preferably from the group consisting of SEQ ID NOS: 6, 9, and 10, and most preferably the amino acid sequence of SEQ ID NO: 6.

The dendritic cell-targeting peptide for treating or preventing cancer binds to dendritic cells in vivo and induces activation of the dendritic cells.

The dendritic cell-targeting peptide for treating or preventing cancer may further include at least one selected from the group consisting of a tumor antigen and flagellin and may include four consecutive copies of the tumor antigen.

The tumor antigen may include the amino acid sequence of SEQ ID NO: 22 and the flagellin may include the amino acid sequence of SEQ ID NO: 23.

The dendritic cell-targeting peptide for treating or preventing cancer may include the amino acid sequence of SEQ ID NO: 6, the amino acid sequence of SEQ ID NO: 22, and the amino acid sequence of SEQ ID NO: 23 and preferably the amino acid sequence of SEQ ID NO: 25.

The dendritic cell-targeting peptide for treating or preventing cancer may induce long-term survival and have an immune potentiating effect.

In addition, the present disclosure provides a pharmaceutical composition for treatment or prevention of cancer, the composition including the dendritic cell-targeting peptide for treating or preventing cancer.

Furthermore, the present disclosure provides a vaccine composition for treatment or prevention of cancer, the composition including the dendritic cell-targeting peptide for treating or preventing cancer.

The pharmaceutical composition or vaccine composition for treatment or prevention of cancer may further include at least one selected from the group consisting of a tumor antigen and flagellin wherein four copies of the tumor antigen may exist in sequence.

The pharmaceutical composition or vaccine composition for treatment or prevention of cancer may include the dendritic cell-targeting peptide, the tumor antigen, and the flagellin in a single integrated peptide or as respective separate peptides.

### Advantageous Effects

The composition containing the novel peptide targeting dendritic cells in vivo, a tumor antigen, and flagellin according to the present disclosure induces potent activation of dendritic cells and immune-potentiating efficacy, which leads to an increased tumor antigen-specific anti-cancer immune response and exhibits outstanding anti-tumor effects.

### Brief Description of the Drawings

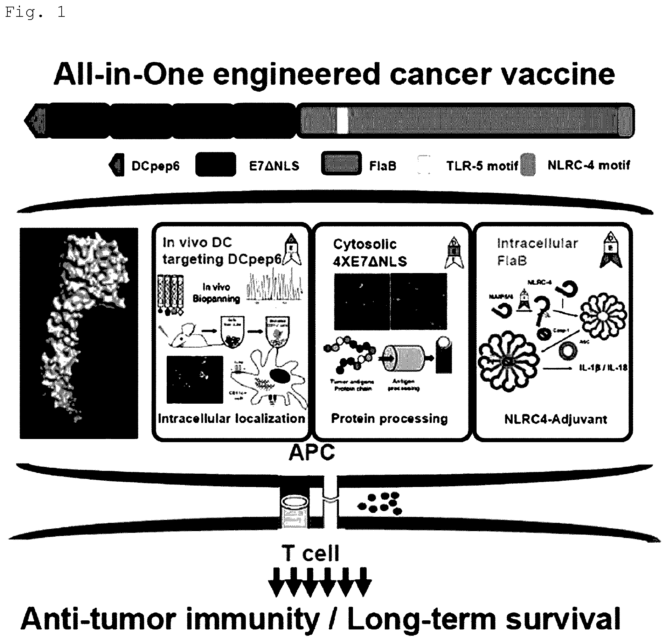

FIG. 1 is a view of the general strategic scheme of the present disclosure.
FIG. 2 is a schematic representation of in vivo biopanning processes in mice.
FIG. 3 shows enrichment numbers and frequencies of phagemid particles by round of in vivo biopanning in mice.
FIG. 4 shows sequences of the peptides obtained according to round of in vivo biopanning in mice.
FIG. 5 shows six peptides selected after two rounds of in vivo biopanning in mice.
FIG. 6 is a graph showing the cellular uptake of the DC-targeting peptides of the present disclosure in bone marrow-derived dendritic cells (BMDCs).
FIG. 7 shows the cellular uptake of DC-targeting peptides of the present disclosure in lymph node cells, splenic DCs, and BMDCs.
FIG. 8 shows the positions of DCpep6 peptide of the present disclosure in cervical lymph nodes (cLN) cells after intranasal administration of the peptide as visualized on confocal microscopic images.
FIG. 9 shows schematic views of the peptide components of the present disclosure.
FIG. 10 is an image of FlaB, E7ΔNLS, EF, and DEF after SDS-PAGE and Western blot analysis.
FIGS. 11 and 12 show tumor volumes and sizes in mice to which E7FL, E7ΔNLS, and FlaB are administered alone or in combination.
FIG. 13 is a plot of TLR5-dependent NF-κB stimulating activity by FlaB, EF or DEF.
FIG. 14 shows the cellular uptake of EF and DEF in BMDCs as measured by flow cytometry.
FIG. 15 shows positions of EF and DEF in BMDCs and Raw264.7 cells as visualized on confocal microscopic images.
FIG. 16 shows biodistribution of EF and DEF in lymph nodes according to time after administration.
FIG. 17 shows measurements for the cellular uptake of EF and DEF in draining inguinal lymph node (iLN) cells.
FIG. 18 and 19 shows survival rates and tumor volumes in mice to which E7 Pep+F, E7ΔNLS, EF, and DEF were administered.
FIG. 20 shows expression levels of CD80 and CD86 in BMDCs treated with EF and DEF.
FIGS. 21 and 22 shows E7-CTL epitope-specific tetramer-positive cells in peripheral blood CD8⁺ from mice injected with E7ΔNLS, EF, and DEF.
FIGS. 23 and 24 shows IFN-γ producing cells in the spleen or tumor lymph nodes from mice injected with E7ΔNLS, EF, and DEF.
FIG. 25 shows survival rates and tumor volumes of wild-type (WT), TLR5-knockout (TLR5^{-/-}), and NLRC4-knockout (NLRC4^{-/-}) mice injected with EF and DEF.
FIG. 26 shows division indices of CD8⁺ T cells in wild-type (WT) and NLRC4-knockout (NLRC4^{-/-}) mice injected with EF and DEF.

### Mode for Carrying Out the Invention

Hereinafter, an exemplary embodiment of the present invention will be described in detail with reference to the accompanying drawings so that those skilled in the art can easily achieve the present invention. As understood by those skilled in the art, the following exemplary embodiment may be modified in various ways without departing from the concept and scope of the present invention.

Throughout the description, unless explicitly described to the contrary, the word "include" and variations such as "includes" or "including" will be understood to imply the inclusion of stated elements but not the exclusion of any other elements.

The present disclosure provides a novel peptide, discovered through biopanning, for targeting dendritic cells in vivo.

With excellent ability to target dendritic cells isolated from the spleen and lymph nodes and various bone marrow-derived cells, the novel in-vivo dendritic cell-targeting peptide can effectively activate dendritic cells in vivo.

In an embodiment, the novel in-vivo dendritic cell-targeting peptide of the present disclosure may include the amino acid sequence of one of SEQ ID NOS: 1 to 11, preferably the amino acid sequence of one of SEQ ID NOS: 3, 4, 6, 9, and 10, more preferably the amino acid sequence of one of SEQ ID NOS: 6, 9, and 10, and most preferably the amino acid sequence of SEQ ID NO: 6.

As used herein, the term "biopanning" refers to an affinity selection technique which selects for peptides binding to a given target from peptide libraries constructed by various display methods such as phage display, bacterial display or mRNA display, ribosome display, yeast display, and the like.

As used herein, the term "dendritic cells" (DCs) refers to professional antigen presenting cells that interact with T cell receptors through the major histocompatibility complex (MHC)/peptide to regulate the activation and differentiation of T cells, thus playing a crucial role in coordinating innate and adaptive immune responses. To prepare human DCs, CD14-positive cells are isolated from human peripheral circulating blood and then differentiated into DCs with the cell identification factors CD11c and CD68 positive.

As used herein, "CD11c", known as complement receptor 4, is a member of the integrin CD18 family. CD11c is in the form of a heterodimer composed of CD11c/CD18 and is involved in phagocytosis. Human CD11c is expressed primarily on the plasma membrane of monocytes, macrophages, natural killer cells, and most dendritic cells. For mice, however, it is almost exclusively expressed in dendritic cells and is thus known as the best marker for these cells.

The present disclosure provides a peptide, targeting dendritic cells, for treating or preventing cancer.

Also, the present disclosure provides a polynucleotide including the nucleic acid sequence coding for the peptide.

In an embodiment, the peptide may include any one selected from the group consisting of the amino acid sequences of SEQ ID NOS: 1 to 11, preferably the amino acid sequences of SEQ ID NOS: 3, 4, 6, 9, and 10, more preferably the amino acid sequences of SEQ ID NO: 6, 9, and 10, and most preferably the amino acid sequence of SEQ ID NO: 6.

The peptide may bind to DCs and induce the activation of the cells in vivo.

The DC-targeting peptide for treating or preventing cancer may further include at least one selected from the group consisting of a tumor antigen and flagellin.

In an embodiment, the peptide may include the amino acid sequence of SEQ ID NO: 6, the amino acid sequence of SEQ ID NO: 22, and the amino acid sequence of SEQ ID NO: 23.

In an embodiment, the peptide may include four consecutive copies of the amino acid sequence of SEQ ID NO: 22 and preferably the amino acid sequence of SEQ ID NO: 25 (DCpep6-4xE7ΔNLS-FlaB).

The amino acid sequence of SEQ ID NO: 22 represents a tumor antigen, and this tumor antigen is a tumor-specific antigen expressed only in tumor cells and may be a protein caused by mutation, a tumor-specific oncogene, or a viral oncogene.

In an embodiment, the tumor antigen may be E7NLS, which is HPV16 E7 (E7_{FL}) with the N-terminal nuclear localization sequence (NLS) removed therefrom.

The amino acid sequence of SEQ ID NO: 23 represents flagellin B (FlaB) derived from *Vibrio vulnificus,* and this flagellin is a major protein that constitutes the filament of bacterial flagella and can induce an immune response in an infected host when flagellated bacteria infect. Specifically, Toll-like receptor 5 (TLR5), which exists on the cell membrane surface of the human body, can interact with the flagellin to cause intracellular signaling which leads to an increase in the transcription factor, resulting in inducing innate immune signaling activation and modulating acquired immune response. Also, the NLR family, apoptosis inhibitory protein (NAIP) existing in human cells can interact with the flagellin to induce the activation of the NLR containing a caspase activating and recruitment domain protein 4 (NLRC4) inflammasome, which leads to an increase in the expression of the transcription factor NF-κB, resulting in activating innate immune signaling and modulating acquired immune responses.

In an embodiment, the flagellin may be flagellin B (FlaB) derived from *Vibrio vunificus,* and may be prepared using the method disclosed in Korean Patent No. 10-0795839, the content of which is hereby incorporated by reference in its entirety.

The present disclosure provides a composition for the treatment or prevention of cancer, the composition including a peptide, targeting dendritic cells in vivo, for treating or preventing cancer.

In addition, the present disclosure a vaccine composition for the treatment or prevention of cancer, the vaccine composition including a peptide, targeting dendritic cells in vivo, for treating or preventing cancer.

The pharmaceutical composition or vaccine composition for the treatment or prevention of cancer, which includes a peptide, targeting dendritic cells in vivo, for treating or preventing cancer, may further include at least one selected from the group consisting of a tumor antigen and flagellin.

The pharmaceutical composition or vaccine composition for the treatment or prevention of cancer, which includes a DC-targeting, cancer treatment or prevention peptide, may include the DC-targeting peptide, the tumor antigen, and the flagellin in a single integrated peptide or as respective separate peptides.

In an embodiment, the pharmaceutical composition or vaccine composition may include a single peptide including all the amino acid sequences of SEQ ID NOS: 6, 22, and 23, or respective separate peptides including the amino acid sequences of SEQ ID NOS: 6, 22, and 23.

In an embodiment, the peptide may include four consecutive copies of the amino acid sequences of SEQ ID NO: 22 and preferably the amino acid sequence of SEQ ID NO: 25 (DCpep6-4xE7ΔNLS-FlaB).

The pharmaceutical or vaccine composition of the present disclosure may include antigen non-specific and specific immune-inducing substances, thereby inducing an overall immune response in vivo and a specific immune response to a particular antigen.

The pharmaceutical or vaccine composition of the present disclosure may exhibit an anti-tumor effect.

The pharmaceutical or vaccine composition of the present disclosure may show an immune-potentiating effect.

The pharmaceutical or vaccine composition of the present disclosure may induce long-term survival of a subject to which the composition is administered.

As used herein, the term "pharmaceutical composition" refers to a composition that is administered for a specific purpose. In the context of the present disclosure, the pharmaceutical composition is a composition, including a peptide targeting DCs in vivo, a tumor antigen, and flagellin, for treating or preventing cancer.

As used herein, the term "vaccine" refers to a biological preparation that induce an immune response against a corresponding pathogen in hosts such as animals including humans, whereby preventing infection or reinfection by the pathogen, reducing the severity of symptoms caused by the pathogen or eliminating the symptoms, or substantially or completely removing the pathogen or the disease caused by the pathogen is achieved. Also, the term "vaccine" used in the present disclosure refers to a biological preparation that can regulates an immune response to a specific antigen in hosts such as animals including humans, to reduce the severity or eliminate the symptoms associated with the disease related to that antigen, or substantially or completely remove the disease. Therefore, the "vaccine composition" of the present disclosure may be administered to animals, including humans, either prophylactically before infection by the pathogen or therapeutically after infection by the pathogen.

As used herein, the term "treatment" means any act which improves or beneficially changes a symptom in a subject that is suspected of or has undergone the onset of disease by administration of the composition, and the term "prevention" means any act that inhibits or delays the development of disease by administration of the composition.

With the ability to effectively not only activate DCs, but also induce tumor cell-specific immunity, the present disclosure can bring about a remarkable improvement in anticancer immunity.

A better understanding of the present disclosure may be obtained through the following examples, which are set forth to illustrate, but are not to be construed to limit, the present disclosure.

### EXAMPLE 1

### In Vivo Biopanning for Discovering Dendritic Cell-Targeting Peptide

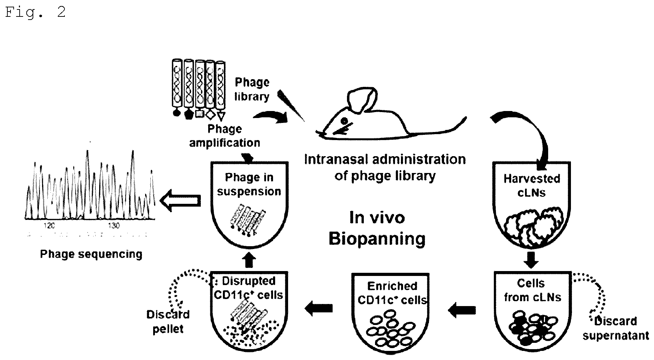

As illustrated in FIG. 2, dendritic cell (DC)-targeting peptides were discovered in vivo as follows.

For in vivo biopanning, 10 µl of phagemid library (10¹² pfu/ml) was given by intranasal instillation into each nostril of Balb/c mice. Library phage solution was instilled into three mice. After 6 hours of in vivo panning, mice were euthanized, and cervical lymph nodes (cLNs) were harvested. The harvested cLNs were centrifuged and washed at least three times in 0.2% PBST, followed by one round of washing in 100 µl of 0.2 M glycine-HCl buffer, pH 2.2. Then, the cLNs were placed in the same buffer and incubated at room temperature for 10 minutes. Glycine buffer was replaced by cold 1x PBS. Capsules of cLNs were broken gently, and the cell suspension was passed through a 40 um cell strainer (Falcon, 352340). The resulting single-cell suspension was centrifuged and treated with glycine buffer for 5 min, after which the cells were washed in PBS and suspended in MACS buffer.

The cell suspension was labeled with CD11c+ MicroBeads MACS (Miltenyi Biotec, 130-052-001), and DCs were purified by application of a magnetic field, as per the manufacturer's recommendations. Purified DCs were subjected to glycine buffer for an additional 5 minutes, after which cells were washed in PBST (1× PBS plus 0.05% TWEEN20) at least three times. Then, to elute the intracellular phage, cells were suspended in 100 µl glycine buffer and disrupted by five freeze thaw cycles with in-between high-speed centrifugations (13000 rpm, 10 minutes). Disruption of cells was confirmed by trypan blue staining. At the end of the cycle, glycine buffer was neutralized with an equal volume of 1x PBS (pH 7.5). The resulting preparation was centrifuged, and the supernatant containing the eluted phage was harvested and stored at -20°C until use. The phage titer in the preparation was estimated by standard protocols. Individual bacterial clones containing phagemid were grown to an OD600 equivalent to 0.5 in Luria broth (LB) containing ampicillin (200 µg/ml). Equal volumes of all 45 bacterial clones were mixed together, and phages were eluted using M13KO7 helper phage for 13 hours and amplified in *E. coli* TG1. Phage from the cell-free supernatant was recovered by PEG/NaCl precipitation (4°C/o/n). Similar to the foregoing (round 1), round 2 (R2) panning was performed using amplified phage harvested after R1. The DNA sequences of phages harvested in each round were determined dideoxy sequencing.

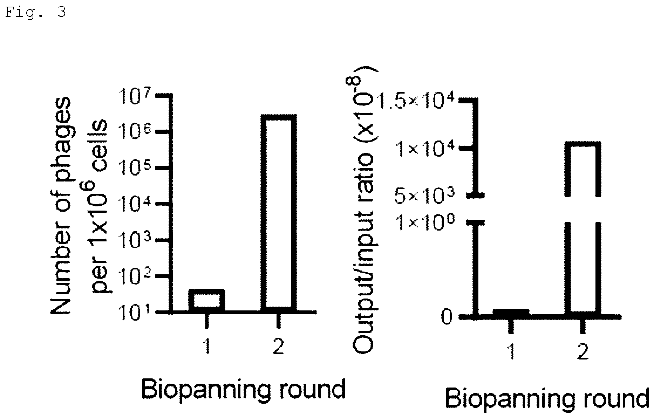

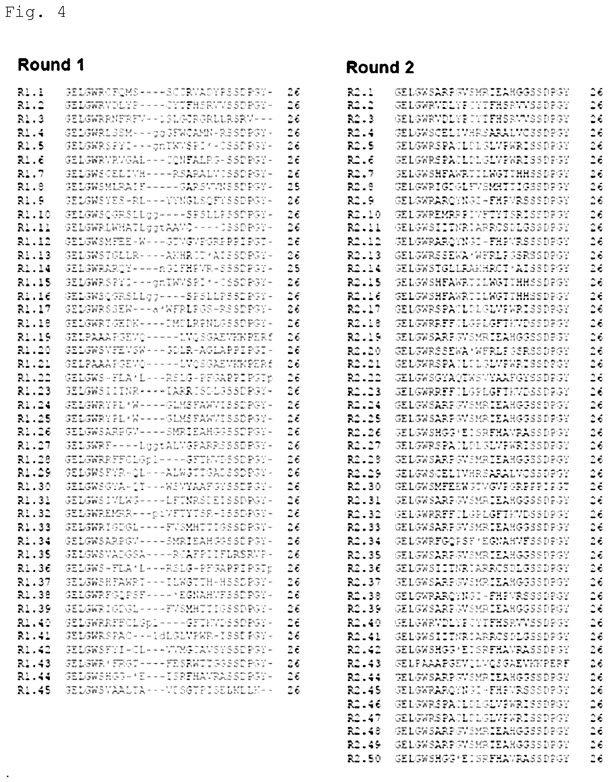

After two rounds of the biopanning, the phage recovery rates according to biopanning round were calculated and are depicted in FIG. 3 and the peptide sequences obtained according to biopanning rounds are presented in FIG. 4.

As shown in FIGS. 3 and 4, after the two rounds of in vivo biopanning, the phage recovery rate was increased while the overall diversity of the peptide sequence was reduced. In addition, repetitively appearing peptide sequences were identified.

Moreover, among the 50 sequenced peptides after the second round, six peptides determined in multiplex manners to test DC-targeting ability were selected and are presented in Table 1, below.

**TABLE 1**

| Peptide No. | Amino Acid Sequence | SEQ ID NO: |
|---|---|---|
| 1 | ARPGVSM**R**IEAHGG | SEQ ID NO: 1 |
| 2 | ARQYNGIFHPV**R**S | SEQ ID NO: 2 |
| 3 | SPACLDLGLVPW**R**I | SEQ ID NO: 3 |
| 4 | VDLYPCYTFHS**R**VV | SEQ ID NO: 4 |
| 5 | HFAW**R**TILWGTTHH | SEQ ID NO: 5 |
| 6 | RFFCLGPLGFTKVD | SEQ ID NO: 6 |

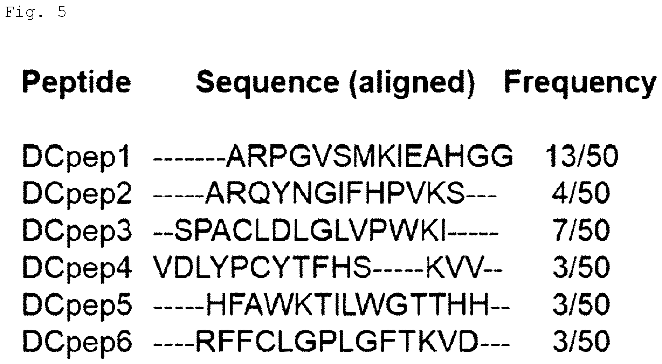

For determining the ability to target DCs, visualization of the peptides is required. To visualize the intracellular peptides, biotin is tagged to the peptides. In addition, biotinylation is made on a lysine residue in the peptides. Since none of peptides 1 to 5 contained lysine, (Lys; K), they were modified to contain lysine in place of arginine (Arg; R) due to similar electrochemical characteristics therebetween. Thus, the six peptides finally determined ((DCpep 1 to 6) are given in Table 2 and FIG. 5.

**TABLE 2**

| Peptide No. | Amino Acid Sequence | SEQ ID NO: |
|---|---|---|
| DCpep1 | ARPGVSMKIEAHGG | SEQ ID NO: 7 |
| DCpep2 | ARQYNGIFHPVKS | SEQ ID NO: 8 |
| DCpep3 | SPACLDLGLVPWKI | SEQ ID NO: 9 |
| DCpep4 | VDLYPCYTFHSKVV | SEQ ID NO: 10 |
| DCpep5 | HFAWKTILWGTTHH | SEQ ID NO: 11 |
| DCpep6 | RFFCLGPLGFTKVD | SEQ ID NO: 6 |

### EXAMPLE 2

### Targeting Efficiency of Dendritic Cell-Targeting Peptide

The 6 peptides determined in Example 1 were measured for DC-targeting efficiency as follows.

Bone marrow (BM) cells were isolated by flushing femurs and tibias of C57BL/6 mice, and red blood cells (RBCs) were depleted by ACK lysis (Gibco, A10492-01). The cells were centrifuged and strained through a 70 um filter before being resuspended in RPMI 1640 supplemented with 10% heat-inactivated FBS, 100 units/ml penicillin, and 100 ug/ml streptomycin. The cells were resuspended in complete RPMI 1640 culture medium containing GM-CSF (10 ng/ml; R&D Systems, 415-ML-010) and IL4 (10 ng/ml; R&D Systems, 415-ML-010), inoculated into a cell culture dish, and cultured at 37°C and 5% CO₂. The culture medium was refreshed every two days. On day 7 or 8, DCs loosely bound to the bottom were collected by gentle pipetting. Splenic DCs were prepared by CD11c+ MicroBeads MACS (Miltenyi Biotec, 130-052-001) following the manufacturer's instructions.

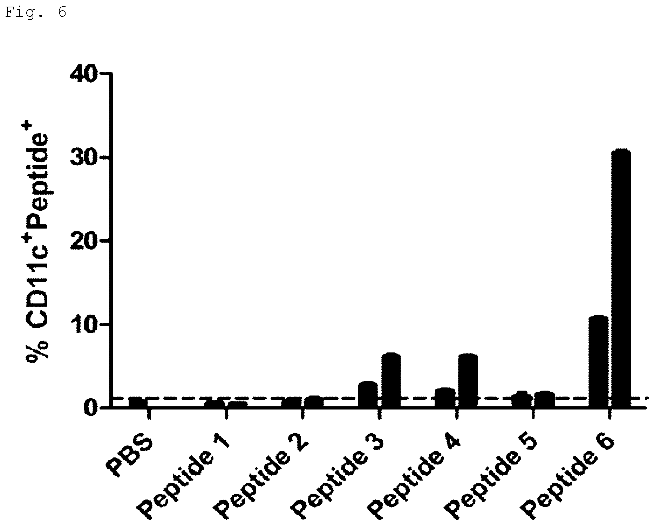

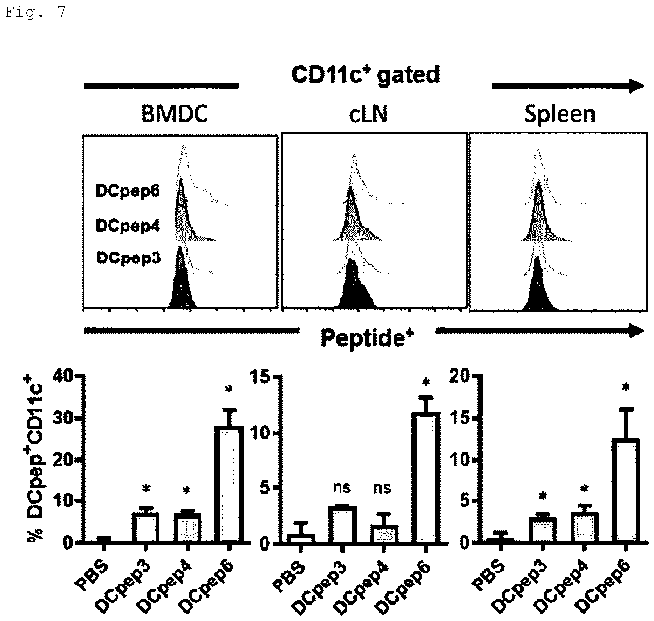

Peptides were synthesized using 9-fluorenylmethylcarbonyl chemistry, purified using highpressure liquid chromatography to more than 95% purity, and biotinylated in the side chain (AnyGen Inc., South Korea). Peptides were dissolved in appropriate solvents according to the manufacturer's instructions to a concentration of 0.5 mg/ml. To test CD11c⁺ cell targeting by the synthesized peptides, cLNs, splenic DCs, and BMDCs were treated with the 6 peptides for 2 hours in RPMI 1640 medium. After incubation with peptides, the cells were harvested, washed and stained with anti-mouse CD11c⁺ APC-conjugated Ab (eBioscience, Clone: N418, 170114-82) and streptavidin-AF488. Cells were FACS analyzed by gating upon the CD11c⁺ cell population, and the results are depicted in FIGS. 6 and 7.

As can be seen in FIG. 6, it was understood from data from the assay for the cellular uptake of the peptides by bone marrow-derived dendritic cells that the targeting ability of DCpep 1, 2, and 5 was similar to that of the negative control whereas DCpep 3, 4, and 6 efficiently targeted CD11c⁺-BMDC, with remarkably higher efficiency for DCpep 6.

In addition, as shown in FIG. 7, the three peptides (DCpep 3, 4, and 6) with excellent efficiency exhibited increased cellular uptake in all of cervical lymph node cells, splenic dendritic cells, and bone marrow-derived dendritic cell. Among others, DCpep6 (SEQ ID NO: 6) exhibited remarkably higher specific signals.

Thus, among the dendritic cell-targeting peptides discovered through biopanning according to the present disclosure, DCpep 3, DCpep 4, and DCpep 6 were found to exhibit excellent targeting efficiency, with the highest signals for DCpep 6 (SEQ ID NO: 6). Furthermore, the peptides were observed to target dendritic cells isolated from lymph nodes and spleen as well as bone marrow-derived dendritic cells.

### EXAMPLE 3

### Targeting Ability of Dendritic Cell-Targeting Peptide

The ability of DCpep6 peptide selected in Example 2 to target dendritic cell in vivo was determined as follows.

The peptide at appropriate concentrations in a total volume of 10 µl/nostril was given intranasally to BALB/c mice. A scrambled peptide previously shown to be nonspecific to DCs was used as a negative control. At 6.5 hours post-inoculation, mice were sacrificed, and their cLNs were harvested. Single-cell suspensions were prepared by gently tearing the LN capsule. After gentle washing in 1x PBS, cells were stained with anti-mouse CD11c⁺ APC-conjugated Ab (eBioscience, Clone: N418, 170114-82) and streptavidin-AF488, and FACS analysis was performed.

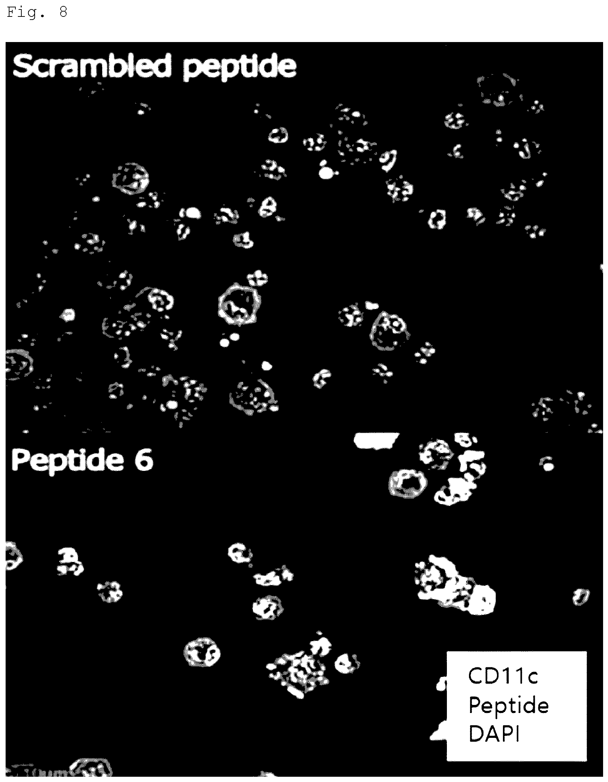

As shown in FIG. 8, DCpep6 was clearly localized in the cytoplasm of CD11c⁺ cells from cervical lymph nodes, compared to the negative control. Therefore, DCpep6 (SEQ ID NO: 6) of the present disclosure, discovered by biopanning, was demonstrated to have ability to target dendritic cells (CD11c⁺).

### EXAMPLE 4

### Production of Peptide

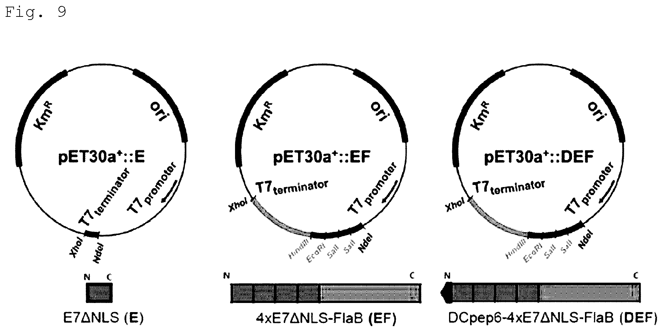

An E7NLS peptide, which is an HPV16 E7 (E7FL) variant with deletion of the N-terminal nuclear localization sequence (NLS), was produced so as to exclude the plausibility of tumorigenesis by HPV16 E7. To this end, pET30a⁺ plasmids (Novagen, 69909 - Merck Millipore) carrying a DNA fragment corresponding to the truncated E7 sequence were constructed, as shown in Table 3 and FIG. 9. The insert DNA fragment was amplified by PCR using a codon-optimized DNA template and the primer set of SEQ IS NOS: 14 and 15.

*Vibrio vunificus*-derived FlaB was produced according to the method disclosed in Lee SE, et al. A bacterial flagellin, Vibrio vulnificus FlaB, has a strong mucosal adjuvant activity to induce protective immunity. Infect Immun. 2006;74(1):694-702.

For use in producing the peptide of 4xE7ΔNLS-FlaB (EF), first, the synthesized 4xE7ΔNLS DNA fragment (NdeI-E7ΔNLS-EcoRI-E7ΔNLS-SalI-E7ΔNLS-SalI-E7ΔNLS-HindIII) was cloned into a pET30a+ plasmid with overhangs recognized by the specific restriction enzymes (REs) of NdeI and HindIII. Next, the DNA fragment of FlaB was generated by PCR using the primer set of SEQ ID NOS: 18 and 19 designed to form HindIII and XhoI overhangs, respectively. The DNA fragment of FlaB was digested by HindIII and XhoI REs and fused to the C-terminus of 4xE7ΔNLS to generate pET30a+::4xE7ΔNLS::FlaB.

DCpep6-4xE7ΔNLS-FlaB (DEF) was generated. In this regard, a DNA fragment of 3xE7ΔNLS::FlaB was cut out from pET30a+::4xE7ΔNLS::FlaB by EcoRI-XhoI REs and cloned into a pET30a+ plasmid to construct pET30a+::3xE7ΔNLS::FlaB. Then, the DNA fragment of DCpep6-E7ΔNLS (NdeI-DCpep6-E7ΔNLS-EcoRI) was amplified by PCR using the primer set of SEQ ID NOS. 16 and 20 designed to form NdeI and EcoRI overhangs, respectively and cloned into an pET30a+::3xE7ΔNLS::FlaB at the N-terminus to produce pET30a+::DCpep6::4xE7ΔNLS::FlaB.

The DNA sequences of the expression vectors were confirmed by the dideoxy-chain termination sequencing method via the Macrogen Online Sequencing Order System (http://dna. macrogen.com/kor/). The resulting plasmid was transformed into competent *Escherichia coli* BL21 cells. Protein expression was induced by incubation with 0.2 mM isopropyl-β-D-thiogalactoside (IPTG) for 18 hours at 20°C, and cells were pelleted by centrifugation and stored at -80 °C until use. The bacterial cell pellets were lysed with 50 ml of lysis buffer (pH 8; 50 mM NaH2PO4, 300 mM NaCl, 10 mM imidazole, 0.1% Triton X-100, 0.1% Tween and 20 µM phenylmethylsulfonyl fluoride). Following centrifugation at 18,000 rpm for 30 minutes, the cell-free supernatant was loaded on a column containing Ni-NTA agarose beads (Qiagen, Hilden, Germany) according to the manufacturer's instructions.

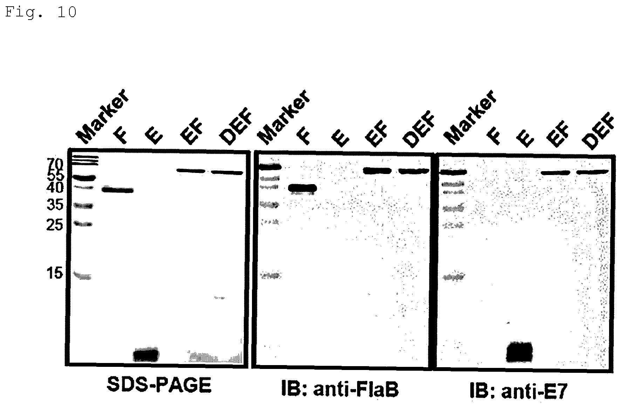

The purity of the recombinant proteins was confirmed by SDS-PAGE and subsequent Western blot analysis with anti-E7 or anti-FlaB antibodies raised in BALB/c mice, and the results are depicted in FIG. 10. Lipopolysaccharide (LPS) contamination was removed by treatment with Triton X-114 (Sigma-Aldrich, St. Louis, MO), and traces of Triton X-114 were removed by incubation with 0.3 g of Bio-Beads^{™} SM-2 (Bio-Rad Laboratories, Inc., Hercules, CA) for 1 ml of protein according to the manufacturer's instructions. The residual LPS content was determined by using a gel-clotting Endosafe LAL kit (Charles River, Charleston, SC, Cat# R15015). The LPS levels in protein preparations were kept below the FDA guidelines (less than 0.15 EU/30 g per mouse).

**TABLE 3**

| Primer | | Base Sequence (5'→3') | SEQ ID NO: |
|---|---|---|---|
| E7 full length (E7_{FL}) | E7-F (NdeI) | GGGAATTCCATATGCATGGTGACACGCCCAC | SEQ ID NO: 12 |
| | E7-R (XhoI) | CCGCTCGAGTGGTTTCTGGGAGCATAT | SEQ ID NO: 13 |
| E7ΔNLS(E) | E7ΔNLS-F (NdeI) | GGGAATTCCATATGATCGATGGGCCGGCCGGT | SEQ ID NO: 14 |
| | E7ΔNLS-R (XhoI) | CCGCTCGAGTGGTTTCTGGGAGCATAT | SEQ ID NO: 15 |
| 4xE7ΔNLS:: FlaB (EF) | E7ΔNLS-F (NdeI) | GGGAATTCCATATGATCGATGGGCCGGCCGGT | SEQ ID NO: 14 |
| | E7ΔNLS-F (EcoRI) | CCGGAATTCAATTGGATCGATGTACGCTA | SEQ ID NO: 16 |
| | E7ΔNLS-R (HindIII) | CCCAAGCTTTGGTTTCTGGGAGCATAT | SEQ ID NO: 17 |
| | FlaB-F (HindIII) | CCCAAGCTTATGGCAGTGAATGTAAATAC | SEQ ID NO: 18 |
| | FlaB-R (XhoI) | CCGCTCGAGGCCTAGTAGACTTAGCGCTG | SEQ ID NO: 19 |
| DCpep6:: 4xE7ΔNLS:: FlaB (DEF) | DCpep6-F (NdeI) | GGGAATTCCATATGCGCTTCTTCTGTTTGGGTCCA | SEQ ID NO: 20 |
| | E7ΔNLS-F (EcoRI) | CCGGAATTCAATTGGATCGATGTACGCTA | SEQ ID NO: 16 |
| | E7ΔNLS-R (HindIII) | CCCAAGCTTTGGTTTCTGGGAGCATAT | SEQ ID NO: 17 |
| | FlaB-F (HindIII) | CCCAAGCTTATGGCAGTGAATGTAAATAC | SEQ ID NO: 18 |
| | FlaB-R (XhoI) | CCGCTCGAGGCCTAGTAGACTTAGCGCTG | SEQ ID NO: 19 |

Peptide sequences of E7_{FL}, E7ΔNLS (E), FlaB (F), 4xE7LNLS::FlaB (EF), and DCpep6::4xE7ΔNLS::FlaB (DEF), prepared by the method, are listed in Table 4, below.

**TABLE 4**

| | Amino acid sequence | SEQ ID NO: |
|---|---|---|
| E7_{FL} | MHGDTPTLHEYMLDLQPETTDLYCYEQLNDSSE EEDEIDGPAGQAEPDRAHYNIVTFCCKCDSTLR LCVQSTVDIRTLEDLLMGTLGIVCPICSQKPLE HHHHHH | SEQ ID NO: 21 |
| E7ΔNLS (E) | MIDGPAGQAEPDRAHYNIVTFCCKCDSTLRLCV QSTHVDIRTLEDLLMGTLGIVCPICSQKPLEHH HHHH | SEQ ID NO: 22 |
| FlaB (F) | MAVNVNTNVAAMTAQRYLNNANSAQQTSMERLS SGFKINSAKDDAAGLQISNRLNVQSRGLDVAVR NANDGISIAQTAEGAMNETTNILQRMRDLSLQS ANGSNSKSERVAIQEEVTALNDELNRIAETTSF GGNKLLNGTYGTKAMQIGADNGEAVMLSLKDMR SDNVMMGGVSYQAEEGKDKNWNVAAGDNDLTIA LTDSFGNEQEIEINAKAGDDIEELATYINGQTD LVKASVGEGGKLQIFAGNNKVQGEIAFSGSLAG ELGLGEGKNVTVDTIDVTTVQGAQESVAIVDAA LKYVDSHRAELGAFQNRFNHAISNLDNINENVN ASKSRIKDTDFAKETTQLTKTQILSQASSSILA QAKQAPNSALSLLG | SEQ ID NO: 23 |
| 4xE7ΔNLS:: FlaB (EF) | MIDGPAGQAEPDRAHYNIVTFCCKCDSTLRLCV QSTHVDIRTLEDLLMGTLGIVCPICSQKPEFID GPAGQAEPDRAHYNIVTFCCKCDSTLRLCVQST HVDIRTLEDLLMGTLGIVCPICSQKPVDIDGPA GQAEPDRAHYNIVTFCCKCDSTLRLCVQSTHVD IRTLEDLLMGTLGIVCPICSQKPVDIDGPAGQA EPDRAHYNIVTFCCKCDSTLRLCVQSTHVDIRT LEDLLMGTLGIVCPICSQKPKLMAVNVNTNVAA | SEQ ID NO: 24 |
| | MTAQRYLNNANSAQQTSMERLSSGFKINSAKDD AAGLQISNRLNVQSRGLDVAVRNANDGISIAQT AEGAMNETTNILQRMRDLSLQSANGSNSKSERV AIQEEVTALNDELNRIAETTSFGGNKLLNGTYG TKAMQIGADNGEAVMLSLKDMRSDNVMMGGVSY QAEEGKDKNWNVAAGDNDLTIALTDSFGNEQEI EINAKAGDDIEELATYINGQTDLVKASVGEGGK LQIFAGNNKVQGEIAFSGSLAGELGLGEGKNVT VDTIDVTTVQGAQESVAIVDAALKYVDSHRAEL GAFQNRFNHAISNLDNINENVNASKSRIKDTDF AKETTQLTKTQILSQASSSILAQAKQAPNSALS LLGLEHHHHHH | |
| DCpep6: : 4xE7ΔNLS: : FlaB (DEF) | MRFFCLGPLGFTKVDIDGPAGQAEPDRAHYNIV TFCCKCDSTLRLCVQSTHVDIRTLEDLLMGTLG IVCPICSQKPEFIDGPAGQAEPDRAHYNIVTFC CKCDSTLRLCVQSTHVDIRTLEDLLMGTLGIVC PICSQKPVDIDGPAGQAEPDRAHYNIVTFCCKC DSTLRLCVQSTHVDIRTLEDLLMGTLGIVCPIC SQKPVDIDGPAGQAEPDRAHYNIVTFCCKCDST LRLCVQSTHVDIRTLEDLLMGTLGIVCPICSQK PKLMAVNVNTNVAAMTAQRYLNNANSAQQTSME RLSSGFKINSAKDDAAGLQISNRLNVQSRGLDV AVRNANDGISIAQTAEGAMNETTNILQRMRDLS LQSANGSNSKSERVAIQEEVTALNDELNRIAET TSFGGNKLLNGTYGTKAMQIGADNGEAVMLSLK DMRSDNVMMGGVSYQAEEGKDKNWNVAAGDNDL TIALTDSFGNEQEIEINAKAGDDIEELATYING QTDLVKASVGEGGKLQIFAGNNKVQGEIAFSGS LAGELGLGEGKNVTVDTIDVTTVQGAQESVAIV DAALKYVDSHRAELGAFQNRFNHAISNLDNINE NVNASKSRIKDTDFAKETTQLTKTQILSQASSS ILAQAKQAPNSALSLLGLEHHHHHH | SEQ ID NO: 25 |

### EXAMPLE 5

### Assay for Efficacy of E7ΔNLS as Tumor Antigen

Female SPF C57BL/6 mice 7 to 8 weeks old were purchased from ORIENT (Seongnam-si, Gyeonggi-do, Korea). TC-1 cells were cultured in RPMI 1640 medium supplemented with 10% heat-inactivated FBS, 100 units/ml penicillin, and 100 ug/ml streptomycin at 37°C under 5% CO₂. Tumors were established in mice by subcutaneous injection of 5 × 10⁴ TC-1 cells in 100 µl of PBS into the right mid-flank of each mouse. When the tumor diameter reached 3-5 mm, the tumor-bearing mice were randomly assigned to groups, anesthetized, and then peritumorally injected with 200 ul PBS, 4 µg FlaB (F), 10 µg E7FL (E_{FL}), 10 µg E7_{FL} + 4 µg FlaB (E_{FL}+F), 8 µg E7ΔNLS, or 8 µg E7ΔNLS + 4 µg FlaB (E+F) three times at regular intervals of five days. Tumor growth was monitored every three days. The tumor volume was calculated with formula V = (tumor length) × (tumor width) × (tumor height)/2.

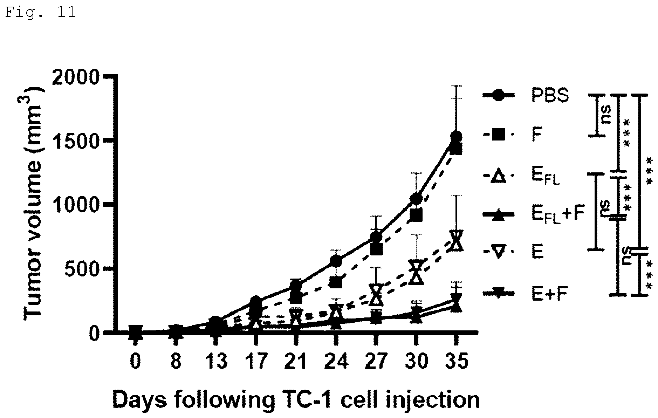

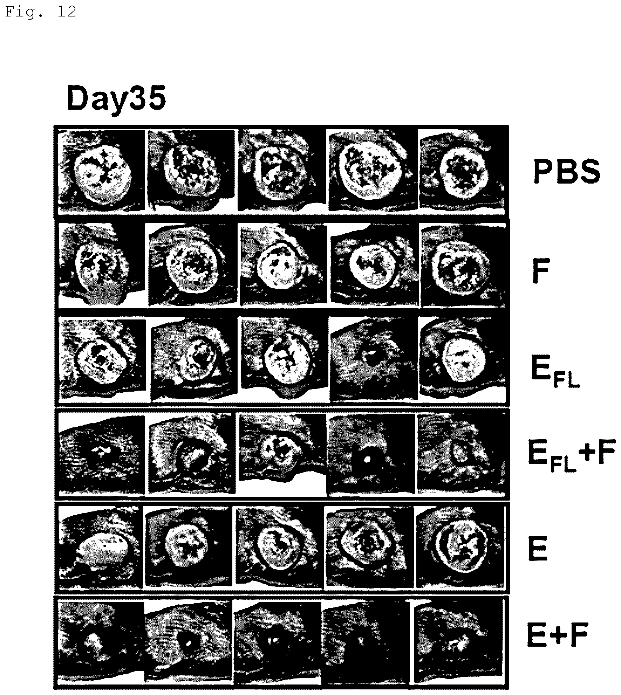

Data of the assay for anti-tumor activity of the peptides are depicted in FIGS. 11 and 12. As can be seen, E7ΔNLS (E) and E7_{FL} (E_{FL}) induced similar levels of tumor suppression and the flagellin (F)-adjuvated groups E7ΔNLS+FlaB (E+F) and E7_{FL}+FlaB (E_{FL}+F) showed significantly enhanced tumor suppression. However, FlaB alone did not manifest any suppressive effect.

These results indicate that E7ΔNLS can serve as an optimal tumor antigen and the flagellin (FlaB) can enhance antigen-mediated tumor suppression.

### EXAMPLE 6

### Assay for TLR5-Stimulating Activity of Peptides

By using HEK-Blue^{™} hTLR5 cells (InvivoGen, hκb-htlr-5) and HEK-Blue^{™} Detection (InvivoGen, hb-det2) assay systems, 4xE7ΔNLS::FlaB (EF) and DCpep6::4xE7ΔNLS::FlaB (DEF) were measured for TLR5-dependent NF-xB-stimulating activity. Also, the EC₅₀ was calculated using triplicate OD 620 nm values for each protein concentration over a wide range of protein concentrations (0.0375 nM-19.29 nM at the AAT Bioquest website).

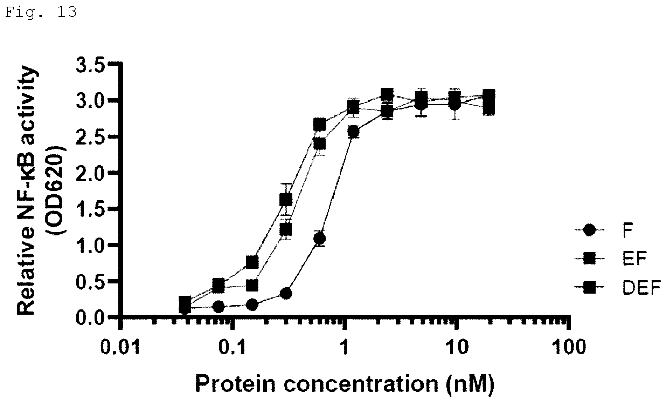

Activity levels of NF-κB were measured and are depicted in FIG. 13. As can be seen, 4xE7ΔNLS::FlaB (EF) and DCpep6::4xE7LΔNLS::FlaB (DEF) increased the activity in dose-dependent manners and thus induced TLR5-stimulating activity. In addition, the EC50 values of 4xE7ΔNLS::FlaB (EF) and DCpep6::4xE7ΔNLS::FlaB (DEF) were 0.38 nM and 0.32 nM, respectively and were much lower than that of FlaB (0.73 nM), suggesting that 4xE7ΔNLS::FlaB (EF) and DCpep6::4xE7ΔNLS::FlaB (DEF) of the present disclosure do not interfere with TLR5-binding activity and stabilize TLR5 binding motifs. Moreover, DCpep6::4xE7ΔNLS::FlaB (DEF) exhibited higher effects, compared to 4xE7ΔNLS::FlaB (EF).

Therefore, DCpep6::4xE7ΔNLS::FlaB (DEF) of the present disclosure, although including structurally or functionally different peptides, was found to stably maintain biological activity.

### EXAMPLE 7

### Assay for Dendritic Cell (CD11c⁺) Targeting Ability of Peptides

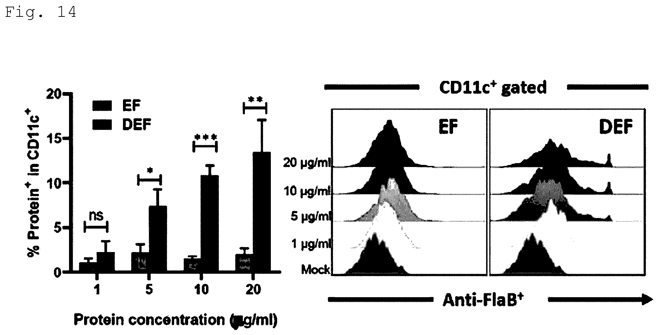

Bone marrow-derived dendritic cells (BMDCs) prepared in the same manner as in Example 2 were treated with the respective concentrations of 4xE7ΔNLS::FlaB (EF) or DCpep6::4xE7ΔNLS::FlaB (DEF) for 2 hours in RPMI 1640 medium. Cells treated with PBS alone were used as a negative control. After incubation, the cells were washed twice with cold 1x PBS containing 3% FBS and then stained with anti-mouse CD11c⁺ antibody (eBioscience, Clone: N418, 25-0114-82) for 1 hour at 4 °C and fixed with 4% paraformaldehyde (T&I, BPP-9004) for 10 minutes at room temperature. The cells were permeabilized using a permeabilization kit according to the manufacturer's instructions (Invitrogen, 00-8333-56) and stained with anti-FlaB antibodies, followed by FACS analysis by gating upon the CD11c⁺ population. The results are depicted in FIG. 14.

In addition, BMDCs or RAW264.7 cells were incubated with 20 µg/ml of 4xE7ΔNLS::FlaB (EF) or DCpep6::4xE7ΔNLS::FlaB (DEF) for 2 hours in RMPI 1640 medium. After incubation, the cells were harvested, washed, fixed with 100% methanol for 15 minutes at room temperature, and stained with anti-mouse CD11c⁺ APC-conjugated antibody and DAPI, a nuclear stain, for 1 hour at room temperature. After two washes with cold 1x PBS, the intercellular distributions of the peptides within the dendritic cells (CD11c⁺) were determined by confocal microscopy (LSM800).

Measurements for the cellular uptake of 4xE7ΔNLS::FlaB (EF) and DCpep6::4xE7ΔNLS::FlaB (DEF) in bone marrow-derived dendritic cells are depicted in FIG. 14. As shown, the concentration-dependent cellular uptake of DCpep6::4xE7ΔNLS::FlaB (DEF) was noted. In contrast to DEF, intracellular 4xE7ΔNLS::FlaB (EF) was scarcely detected.

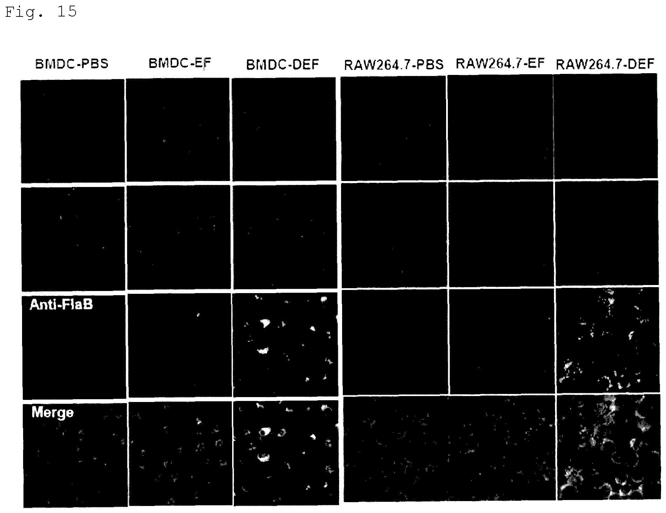

In addition, as shown in FIG. 15, DCpep6::4xE7ΔNLS::FlaB (DEF) was located in the cytoplasm of CD11c⁺ cells and accumulated in a punctuated pattern.

Therefore, possessing the DC-targeting peptide (DCpep6), DCpep6::4xE7ΔNLS::FlaB (DEF) of the present disclosure has excellent ability to target DCs (CD11c⁺).

### EXAMPLE 8

### In vivo Distribution of Peptides

Purified 4xE7ΔNLS::FlaB (EF) or DCpep6::4xE7ΔNLS::FlaB (DEF) was mixed with FNR675-NHS ester (BioActs, Korea) at 4°C and maintained overnight in the dark with stirring. The labeled EF-FNR675 or DEF-FNR675 was then separated from the unconjugated dye using a centrifugal filter (10 kDa cutoff) (Amicon Ultra-4, UFC801024), followed by washing in PBS. Next, the conjugated proteins were quantitatively determined from the calibration curve of the FNR675-NHS ester using a UV-Vis spectrophotometer (UV-2700, Shimadzu, Japan).

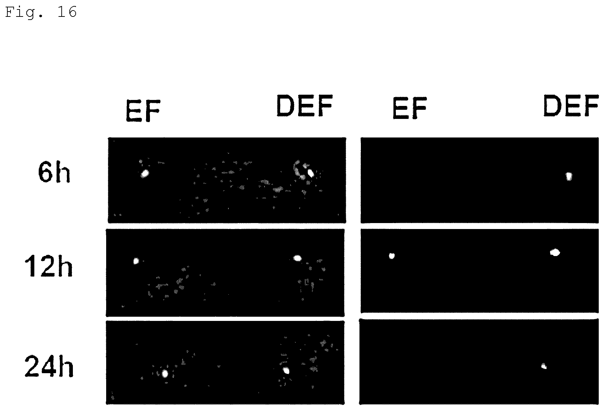

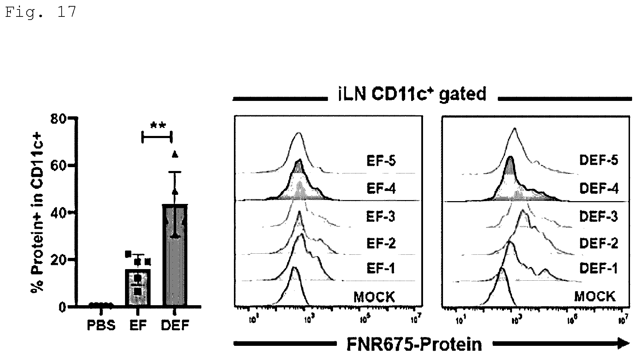

C57BL/5 mice were subcutaneously injected with PBS, 50 µg EF-FNR675, or 50 µg DEF-FNR675 in the inguinal region, and draining inguinal LNs (iLNs) were isolated at 1, 6, 12, and 24 hours post injection. The fluorescence signals in the draining lymph nodes were determined and are depicted in FIG. 16. In addition, six hours after injection, the draining inguinal LN (iLN) cells were prepared, and then in vivo DC-targeting was determined by FACS analysis gating upon CD11c⁺ cell population, and the results are depicted in FIG. 17.

As shown in FIG. 16, a fluorescent signal was detected in the DEF-FNR675 (DEF)-administered mice 6 hours after administration. The fluorescent signal was more intensified at 12 hour and dissipated at 24 hours. Lower fluorescent signals were detected for EF-FNR675 (EF) than DEF-FNR675 (DEF).

In addition, as shown in FIG. 17, the fluorescent signal was detected at higher intensity upon injection of DEF-FNR675 (DEF) and also detected in CD11c+ cells six hours after injection.

Therefore, it was confirmed that the DCpep6::4xE7ΔNLS::FlaB (DEF) of the present disclosure, when administered in vivo, can not only efficiently reach the draining lymph nodes through lymphatic circulation but also interact with dendritic cells (CD11c⁺) in vivo. Moreover, the DEF introduced in vivo was removed from the draining lymph nodes within 24 hours post administration.

### EXAMPLE 9

### Assay for Antitumor Efficacy of Peptides

The tumor-bearing mice were administered the all-in-one vaccine of the present disclosure as in Example 5 and measured for survival rate and tumor volume in the same manner. For a positive control, a group of mice was immunized with HPV16 E7 CTL peptide (amino acid 49-57: RAHYNIVTF), known as a cytotoxic T lymphocyte (CTL)-inducing peptide.

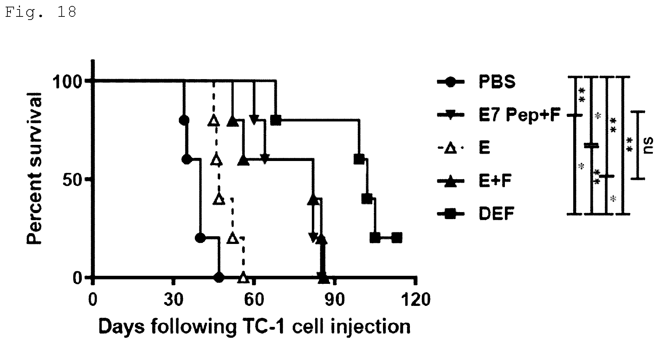

As shown in FIG. 18, E7ΔNLS + FlaB (E+F)- and DCpep6-4xE7ΔNLS-FlaB (DEF)-administered groups showed significantly longer survival than the E7ΔNLS (E)-administered group, with the longest survival for DCpep6-4xE7ΔNLS-FlaB (DEF) injection. In addition, the DEF group exhibited notably longer survival than the HPV16 E7 CTL peptide + FlaB (E7 Pep+F) group.

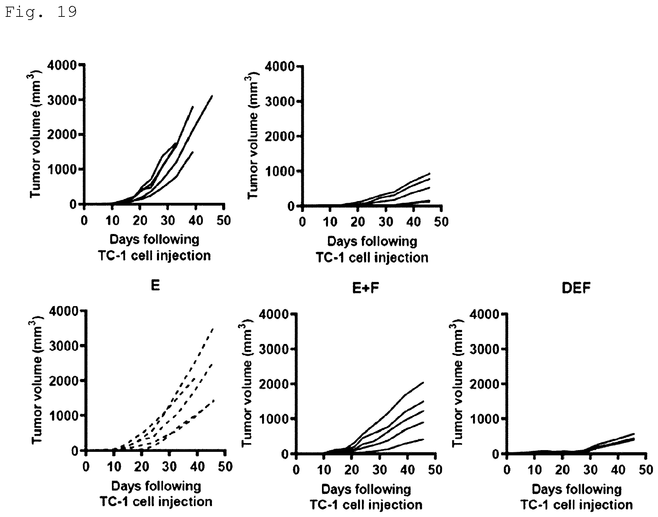

In addition, as shown in FIG. 19, the tumor volume in the DCpep6-4xE7ΔNLS-FlaB (DEF)-administered group was the smallest and remarkably much smaller than that in the HPV16 E7 CTL peptide + FlaB (E7 Pep+F)-administered group.

Therefore, DCpep6::4xE7ΔNLS::FlaB (DEF) of the present disclosure was observed to have an excellent in-vivo antitumor activity as well as enable survival for a long period of time. Furthermore, the peptide including the DC-targeting peptide (DCpep6), the tumor antigen (E7ΔNLS), and the flagellin (FlaB) according to the present disclosure more superb antitumor activity than the formulation including the tumor antigen plus flagellin only.

### EXAMPLE 10

### Assay for Immune Response of Peptide

### 10-1. Antigen-presenting cell activation

The bone marrow-derived dendritic cells (BMDCs) prepared as in Example 2 were treated with PBS, 0.5 µg/ml 4xE7ΔNLS::FlaB (EF), or 0.5 µg/ml DCpep6::4xE7ΔNLS::FlaB (DEF) for 24 hours. The cells were stained on ice with fluorophore-labeled antibodies before flow cytometry analysis.

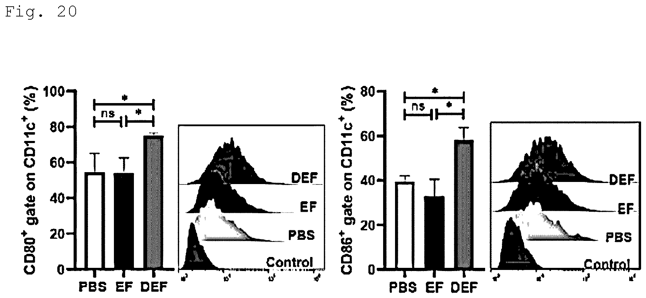

Expression levels of CD80 and CD86 on the BMDC surface were measured and the results are depicted in FIG. 20. As seen, DCpep6::4xE7ΔNLS::FlaB (DEF) induced significantly enhanced expression of CD80 and CD86 molecules, with no statistical significance for the 4xE7ΔNLS::FlaB (EF)-induced expression of CD80 and CD86.

Therefore, DCpep6::4xE7ΔNLS::FlaB (DEF) of the present disclosure was found to activate antigen presenting cells (APCs).

### 10-2. Antigen-specific T cell immune response

The peptides of the present disclosure as in Example 5 were administered to mice with or without tumors and peripheral blood was collected therefrom. After removal of RBC by RBC lysis buffer, the blood was stained with the tetramer antibody (PE-conjugated HPV16 H-2Db-RAHYNIVTF, TB-5008-1, MBL) and CD8 antibody and analyzed by flow cytometry.

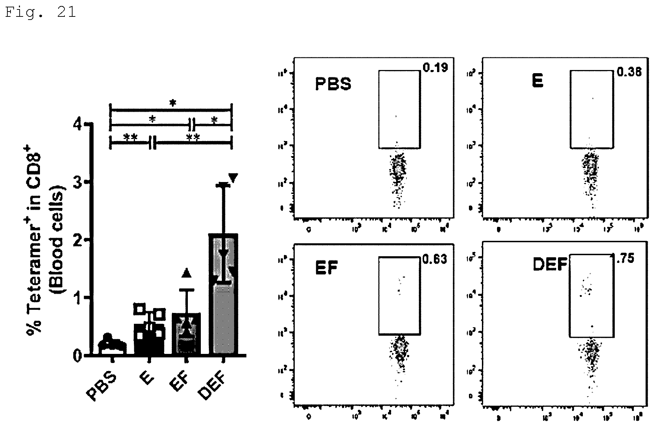

Cytotoxic T lymphocyte (CTL) epitope-specific tetramer+ CB8+ cells in the peripheral blood from the tumor-bearing mice were measured and the results are depicted in FIG. 21. As can be seen, the DCpep6::4xE7ΔNLS::FlaB (DEF)-administered group showed significantly higher levels of the tetramer-positive cells than PBS-, E7ΔNLS (E)- or 4xE7ΔNLS::FlaB (EF)-administered group.

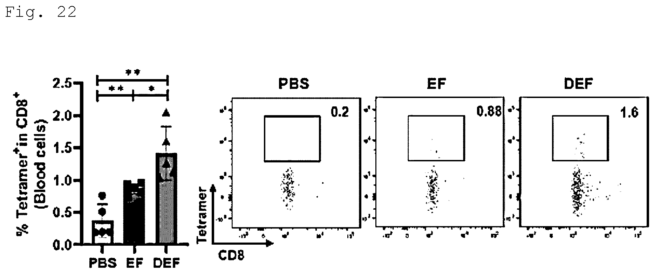

In addition, as shown in FIG. 22, even mice without tumors exhibited remarkably high levels of the tetramer-positive cells when injected with DCpep6::4xE7ΔNLS::FlaB (DEF) .

Therefore, DCpep6::4xE7ΔNLS::FlaB (DEF) of the present disclosure was found to effectively induce antigen-specific T cell immune responses.

### 10-3. Cytotoxic T lymphocyte (CTL) peptide-specific immune response

Single-cell suspensions from the spleen (SPL) or tumor-draining lymph nodes (TDLNs) were prepared by administering the peptides of the present disclosure to mice with or without tumors in the same manner as in Example 5. A total of 1 × 10⁶ SPL or 2.5 × 10⁵ TDLNs cells were seeded into 96-well Filtration ELISpot plates (Merck, HAMAS4510) and stimulated with 1 µg/ml E7 CTL peptide (amino acids 49-57: RAHYNIVTF). Cells stimulated with 10 ng/ml concanavalin A were used as a positive control. After two days of culture of SPL cells or five days of culture of TDLN cells, IFN-γ-producing cells were detected by using the mouse IFN-γ ELISpot Set (BD Bioscience, 551083) according to the manufacturer's instructions. IFN-γ-producing cells were analyzed using a CTL-ImmunoSpot Analyzer and ImmunoSpot Professional Software version 5.0 (Cellular Technology, Shaker Heights, OH, USA).

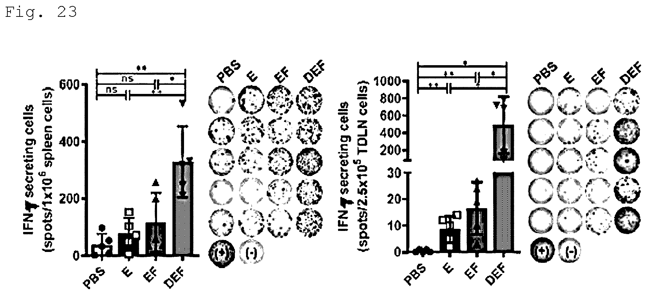

IFN-γ production in the spleen (SPL) and tumor draining lymph nodes (TDLNs) in tumor-bearing mice were measured and the results are depicted in FIG. 23. As seen, immunization with DCpep6::4xE7ΔNLS::FlaB (DEF) group significantly enhanced the IFN-γ production level, compared to PBS, E7ΔNLS (E), and 4xE7ΔNLS::FlaB (EF).

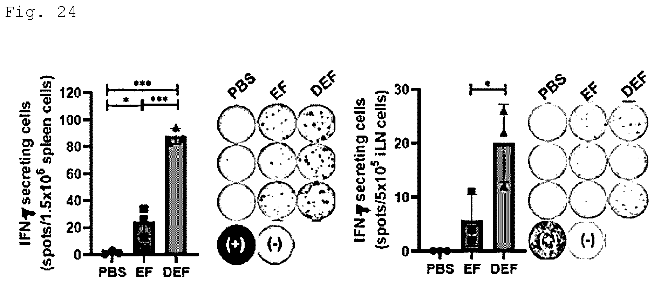

In addition, as shown in FIG. 24, even the mice without tumors were observed to produce a significantly higher level of IFN-γ upon DCpep6::4xE7ΔNLS::FlaB (DEF) immunization.

Therefore, it was confirmed that DCpep6: :4xE7ΔNLS: :FlaB (DEF) of the present disclosure effectively induces CTL peptide-specific immune responses.

Collectively, DCpep6::4xE7ΔNLS::FlaB (DEF) of the present disclosure was found to effectively induce an antigen-specific immune response in vivo and elicit an excellent antitumor immune response through the modulation of immune activity.

### EXAMPLE 11

### Assay for Signaling Pathway of Peptide-Mediated Immune Response

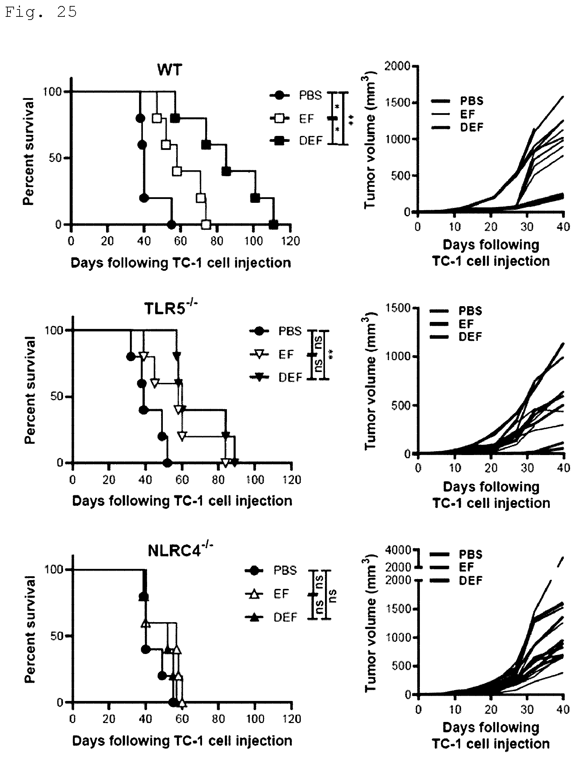

Wild-type (WT), TLR5-knockout (TLR5^{-/-}) and NLRC4-knockout (NLRC4^{-/-}) mouse groups were implanted with TC-1 cells in the right mid-flank. When the tumor size reached approximately 3-5 mm in diameter, the tumor-bearing mice were subcutaneously vaccinated with 200 ul of PBS only, 20 µg of 4xE7ΔNLS-FlaB (EF), 20 µg of Dcpep6-4xE7ΔNLS-FlaB (DEF) in the peritumoral region three times at regular intervals of five days, and the tumor volume and survival of the tumor-bearing mice were measured. The results are depicted in FIG. 25. In addition, TLR5 or NLRC4 knockout did not influence TC-1 tumor growth as analyzed in pre-tests.

As shown in FIG. 25, DCpep6: :4xE7ΔNLS: :FlaB (DEF) immunization in TLR5-knockout (TLR5^{-/-}) mice induced significantly longer survival and decreased the tumor volume, compared to the PBS group, but at significantly lower levels than in the wild-type mice. The effects of DCpep6: :4xE7ΔNLS::FlaB (DEF) on tumor volume and survival were abolished in NLRC4-knockout (NLRC4^{-/-}) mice.

Therefore, the data implies that the adjuvant function in DCpep6::4xE7ΔNLS::FlaB (DEF) would have been mainly executed through NLRC4 inflammasome-mediated activation.

### EXAMPLE 12

### Assay for Peptide-Mediated CD8⁺ T Cell Activation

The bone marrow-derived dendritic cells (BMDCs) prepared in the same manner as in Example 2 were activated for 24 hours with 1 µg/mL 4xE7ΔNLS::FlaB (EF) or DCpep6::4xE7ΔNLS::FlaB (DEF). CD8⁺ splenocytes from TC-1 tumor-bearing mice were prepared using the MagniSort^{™} Mouse CD8 T Cell Enrichment kit (Invitrogen, 8804-6822) according to the manufacturer's instructions, and were labeled with 5 µM CFSE. EF- or DEF-pulsed BMDCs were cocultured with CFSE-stained CD8⁺ cells at a ratio of 1:5 (BMDC:CD8⁺ T cells) for 3 days in RPMI 1640 medium supplemented with 10% fetal bovine serum (HyClone, Logan, UT), 1% penicillin/streptomycin (Life Technologies, Grand Island, NY), and 50 µM 2-mercaptoethanol (Sigma, 516732). CD8⁺ T cell proliferation was assessed by CFSE dilution using flow cytometry.

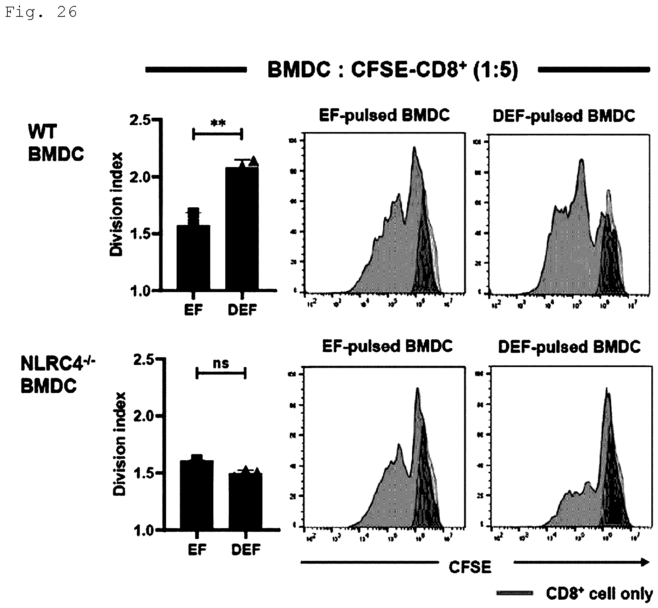

As shown in FIG. 26, DCpep6::4xE7ΔNLS::FlaB (DEF)-pulsed cells in wild-type (WT) mice significantly enhanced the division index of CD8⁺ T cells from tumor-bearing mice, but the division index was rather decreased in NLRC4-knockout (NLRC4^{-/-}) mice.

Therefore, the DCpep6::4xE7ΔNLS::FlaB (DEF) of the present disclosure was discovered to activate CD8⁺ T cells via the NLRC4-mediated inflammasome signaling pathway.

## Claims

1. A dendritic cell-targeting peptide for treatment or prevention of cancer, the peptide comprising an amino acid sequence selected from the group consisting of SEQ ID NOS: 1 to 11.

2. The dendritic cell-targeting peptide of claim 1, comprising any one selected from the group consisting of the amino acid sequences of SEQ ID NO: 3, SEQ ID NO: 4, SEQ ID NO: 6, SEQ ID NO: 9, and SEQ ID NO: 10.

3. The dendritic cell-targeting peptide of claim 1, comprising any one selected from the group consisting of the amino acid sequences of SEQ ID NO: 6, SEQ ID NO: 9, and SEQ ID NO: 10.

4. The dendritic cell-targeting peptide of claim 1, comprising the amino acid sequence of SEQ ID NO: 6.

5. The dendritic cell-targeting peptide of claim 1, wherein the peptide binds to dendritic cells in vivo and induces activation of the dendritic cells.

6. The dendritic cell-targeting peptide of claim 1, further comprising at least one selected from the group consisting of a tumor antigen and flagellin.

7. The dendritic cell-targeting peptide of claim 6, comprising four consecutive copies of the tumor antigen.

8. The dendritic cell-targeting peptide of claim 6, wherein the tumor antigen comprises the amino acid sequence of SEQ ID NO: 22.

9. The dendritic cell-targeting peptide of claim 6, the flagellin comprises the amino acid sequence of SEQ ID NO: 23.

10. The dendritic cell-targeting peptide of claim 6, comprising the amino acid sequence of SEQ ID NO: 6, the amino acid sequence of SEQ ID NO: 22, and the amino acid sequence of SEQ ID NO: 23.

11. The dendritic cell-targeting peptide of claim 6, comprising the amino acid sequence of SEQ ID NO: 25.

12. The dendritic cell-targeting peptide of claim 6, inducing long-term survival.

13. The dendritic cell-targeting peptide of claim 6, having an immune potentiating effect.

14. A polynucleotide, encoding the peptide of any one of claims 1 to 11.

15. A pharmaceutical composition for treatment or prevention of cancer, the composition comprising the peptide of claim 1.

16. The pharmaceutical composition of claim 15, further comprising at least one selected from the group consisting of a tumor antigen and flagellin.

17. The pharmaceutical composition of claim 16, comprising four copies of the tumor antigen.

18. The pharmaceutical composition of claim 16, comprising the peptide of claim 1, the tumor antigen, and the flagellin in a single integrated peptide or as respective separate peptides.

19. A vaccine composition for treatment or prevention of cancer, the composition comprising the peptide of claim 1.

20. The vaccine composition of claim 19, further comprising at least one selected from the group consisting of a tumor antigen and flagellin.

21. The vaccine composition of claim 20, comprising four copies of the tumor antigen.

22. The vaccine composition of claim 20, comprising the peptide of claim 1, the tumor antigen, and the flagellin in a single integrated peptide or as respective separate peptides.
